(19) Europäisches Patentamt
European Patent Office
Office européen des brevets

(11) **EP 2 567 702 A1**

(12) **EUROPEAN PATENT APPLICATION**

(43) Date of publication:
**13.03.2013 Bulletin 2013/11**

(21) Application number: **11007401.0**

(22) Date of filing: **12.09.2011**

(51) Int Cl.:
*A61K 33/00* (2006.01)     *A61K 9/16* (2006.01)
*A61P 35/00* (2006.01)     *A61P 31/10* (2006.01)
*A61P 31/04* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
• **Johannes-Gutenberg-Universität Mainz**
  **55122 Mainz (DE)**
• **Universitätsmedizin der**
  **Johannes Gutenberg-Universität Mainz**
  **55131 Mainz (DE)**

(72) Inventors:
• **Langguth, Peter**
  **63599 Biebergemünd (DE)**
• **Buch, Karl**
  **55127 Mainz (DE)**
• **Nawroth, Thomas**
  **55450 Langenlonsheim (DE)**
• **Schmidberger, Heinz**
  **55270 Essenheim (DE)**

(74) Representative: **Patentanwälte Bitterich, Dr. Keller, Schwertfeger**
  **Westring 17**
  **76829 Landau (DE)**

(54) **A particulate system comprising lanthanides for use in diminishing cell growth/inducing cell killing**

(57)     The Invention relates to a particulate system for use in diminishing cell growth, in particular the growth of cancer cells, comprising one or more water soluble lanthanide compounds that are embedded in a solid biodegradable polymer particle, the polymer being selected from the group consisting of polycarbonic acids, polylactic acids, polyglycolic acids, polypeptides or combinations thereof.

EP 2 567 702 A1

## Description

The finding of appropriate therapeutic treatments of diseases such as bacterial or fungal infections, or cancer is still a major task in the field of medicine and pharmacology. Different approaches have been established over time to be utilized in the treatment of these diseases or to minimize their symptoms. In Europe nearly every third man suffers from cancer during the course of his life. Upon diagnosis of cancer, the survival rate within a term of five years is approximately 55 %. In Germany, roughly 400,000 new cases of patients that suffer of cancer are accounted per year. The most frequent type of cancers among the human population is breast cancer, intestinal cancer and lung cancer. These types of cancer are main targets for different medical treatment approaches. Cancer treatments generally include resections of tumor tissue, chemotherapy with cytostatics and angiogenesis inhibitors. In addition, irradiation therapy is supplied in combination with the application of radio pharmaceuticals, X-rays, thermionic irradiation and neutron irradiation.

One problem associated with the application of irradiation can be seen in the high doses required in order to diminish cell growth of treated cancer cells. The high doses of irradiation however cause a number of severe side effects with unpleasant outcome for the patient. One major problem of such irradiation treatment is that not only the cancer tissue is affected by irradiation but also surrounding healthy tissue. It is therefore an aim in radiology to decrease the irradiation doses required for treatment of diseases. Irradiation enhancers were found to maximize the irradiation effects, and thereby minimizing the doses required for diminishing cell growth. Such enhancers are able to achieve a reduction of the irradiation doses utilized for the treatment of target cells.

In the US 6,770,020 B2 a method of using gadolinium-containing compounds as agents for neutron capture therapy to treat neoplastic cell growth is described. The subject is exposed to a gadolinium-containing compound for a time sufficient to allow the compound to accumulate in neoplastic cells. The subject is then exposed to a thermal and/or epithermal neutron flux, thereby initiating a neutron capture reaction in the gadolinium atoms that results in specific death of neoplastic cells.

In the US 5,888,997 irradiation sensitizers and the use of texaphyrins for irradiation sensitization and other conditions for which X-ray irradiation has proven to be therapeutically effective is described.

T. Nawroth, et al., SRMS 4, Conference "Synchrotron Irradiation in Material Sciences", Grenoble, August 23-25, 2004, describes magnetic liposomes and trapping target hollow magnetic particles for biomedical applications. The method described is used for imaging, and neutron and photodynamic X-ray therapy of cancer.

Against this background, it is object of the present invention to provide an irradiation enhancer system which diminishes the growth of target cells and allows for minimizing the dose of irradiation applied to the target cells in order to minimize the risks of side effects and to increase the efficiency of irradiation treatment.

This object is solved by a particulate system with the technical features of claim 1. The sub claims relate to preferred embodiments of the present invention.

The present invention provides new compounds, which specifically exhibit their effect in tumor tissue, whereas the toxicity to healthy tissue is minimized.

The particulate system according to the present invention comprises one or more water soluble lanthanide compounds (including their salts) that are embedded in a solid biodegradable polymer particle for delivery to the target cells. Preferred polymers used for embedding the lanthanide compound or a mixture thereof are polycarbonic acids, polylactic acids, polyglycolic acids, polypeptides or combinations thereof.

The lanthanide compound is preferably selected from the group consisting of lanthanum, cerium, praseodymium, neodymium, promethium, samarium, europium, gadolinium, erbium, dysprosium, holmium, erbium, thulium, yterbium, lutetium, scandium, yttrium. The use of lanthanide salts, preferably acetate salts, is preferred.

Lanthanide compounds that show a characteristic irradiation pattern upon excitation are suitably detectable by well known analysis methods in the art. In one embodiment, erbium acetate is a preferred lanthanide compound to be packed in a solid biodegradable polymer particle. It is further possible to combine one or more lanthanide compounds or their salts with other types of irradiation enhancers (e.g. cisplatin, resveratrol, hydroxychalcone, roscovitine, amrubicine, amrubicinol) or even cytostatics such as doxorubicine or Paclitaxel.

The polymer of the particulate system is preferably selected from the group consisting of all optically active (D-/L-/DL-) forms of poly(glycolic acid) (PGA), poly (lactic acid) (PLA), poly (lactide-co-glycolide) copolymers (PLGA), polydioxanone (PDS), polyacrylates, polyketales, polycyanoacrylates, polyorthoesters, polyacetates, poly($\varepsilon$ caprolactone), polyphosphozene, polycarbonates, polypeptides, polyiminocarbonates, poly($\beta$-hydroxyester).

Poly(glycolic acid) (PGA), poly(lactic acid) (PLA) and their copolymers are preferred biodegradable polymers of the particulate system according to the present invention. These polymers degrade in the body by hydrolysis of the ester backbone to non-harmful and non-toxic compounds. The degradation products are either excreted by the kidneys or eliminated as carbon dioxide and water through well-known biochemical pathways. The polymers PGA, PLA, PLGA and PDS as well as their copolymers can be used in all optically active forms or as part of a racemic mixture of their active L- and D-forms.

The life time of polymers within the human or animal body and therefore their effectiveness can be controlled by selecting different appropriate end-groups. Preferably, the polymer utilized in the particulate system of the invention has either a free carboxylic acid end group, an ester terminated end group or an alkyl ester end group. Polymers kept with ester terminated and alkyl ester groups typically show longer degradation life times than the free carboxylic analogues. One preferred polymer of the invention is poly(D,L-lactide-co-glycolide) with a free carboxylic acid end group.

The packaging of lanthanides/lanthanide compounds in a solid biodegradable polymer particle surprisingly results in a rather high enrichment of the irradiation enhancer within the particle and hence high delivery doses to the target cells. The use of lanthanide compounds in form of their acetate salts is preferred since there appears to be an unexpected and surprising molecular interaction between chemical residues of the acetate salt and structures of the polymer. The polymer particles provide a high efficiency in the uptake of the lanthanide compounds by the target cells. The polymer particle of the invention is thus a suitable means to deliver the lanthanide compounds to the target cell (e.g. bacterial, fungal or cancer cell).

One major advantage of the invention is that the particulate system uses polymer particles that are biodegradable. The use of biodegradable polymers avoids an unwanted accumulation of polymer compounds within the treated tissue, in particular in the human or animal body. The biodegradable polymer used in the particulate system of the invention will be physiologically degraded after a certain time.

The particulate system of the invention, consisting of lanthanide compounds embedded in solid biodegradable polymers is preferably produced by solvent evaporation. A defined amount of polymer is diluted in dichloromethane. A lanthanide salt (e.g. erbium acetate) is diluted at high concentration in water resulting in an aqueous phase. The aqueous phase is emulsified within the oily phase of the polymer fraction by treatment with an ultrasonic stirrer on ice. The resulting O/W emulsion is transferred into a W/O/W emulsion by addition of approximately 2.5 x vol of a 1 % aqueous solution of polyvinyl alcohol with an average molecule mass of 72,000 g/mol. A subsequent ultrasonic treatment is followed. The resulting emulsion is stirred slowly in 3 x vol of water, preferably in a round-bottomed flask on a magnetic stirrer at low pressure (approximately 500 mbar) for several hours. Following incubation, the solvent dichloromethane is allowed to enter into the aqueous phase and subsequently into the gas phase. The evaporation of the solvent results in a hardening of the polymer particles and their separation. Upon evaporation of the solvent, the size of the produced particles can be controlled by dynamic light scattering (DLS).

The invention further relates to a method for diminishing cell growth, comprising the steps of exposing cells to a particulate system, comprising one or more water soluble lanthanide compounds as irradiation enhancer, wherein the lanthanide compounds are embedded in a biodegradable polymer particle as carrier, the polymer being selected from the group consisting of polycarbonic acids, polylactic acids, polyglycolic acids, polypeptides or combinations thereof, and exposing the cells that are treated with the particulate system to irradiation at a wave length that results in an excitation of the lanthanide compound(s). The method can be used both for *in-vitro* and *in-vivo* treatments. The carrier systems and the methods according to the invention can be used both for therapeutic and diagnostic purposes.

Depending on the lanthanide used, the irradiation dose for treating the target cells is at least 4 Gy. A suitable pre-incubation time for the irradiation treatment of the target cells that were exposed to lanthanide loaded polymer particles is at least 24 h.

The invention further relates to a method for producing particles, comprising one or more water soluble lanthanide compounds that are embedded in a solid biodegradable polymer particle, the polymer being selected from the group consisting of polycarbonic acids, polylactic acids, polyglycolic acids, polypeptides or combinations thereof by incubating the polymer with the lanthanide compound in a suitable solvent solution, emulsifying the polymer/lanthanide mixture and applying solvent evaporation to hardening the particles.

The invention also relates to a pharmaceutical composition, comprising one or more water soluble lanthanide compounds that are embedded in a solid biodegradable polymer particle, the polymer being selected from the group consisting of polycarbonic acids, polylactic acids, polyglycolic acids, polypeptides or combinations thereof for use in the treatment of a disease.

In a preferred embodiment, the disease is a bacterial or fungal infection or cancer. The systems and methods according to the invention can be both applied to prokaryotic and eukaryotic cells. Pathological diseases that are caused by a bacterial or fungal infection are based on pathogenic bacteria or fungi. Both bacterial and fungal cells can be exposed to the particulate system according to the invention. Cell growth is inhibited or reduced in these cells by delivering the lanthanide compounds by the polymer carrier to the respective target cells. The systems and methods according to the invention are also suitable for treatment of pathogenic eukaryotic cells, in particular cancer cells. Cancer tissue / cells can be efficiently treated with polymer particles that are loaded with lanthanide compounds according to the present invention, thereby causing a dose-dependent reduction of cell growth or cell death.

The manufacture and applicability of the particulate system according to the invention is more fully explained in the following. The experimental data that support and demonstrate the present invention are shown in the accompanying Figures.

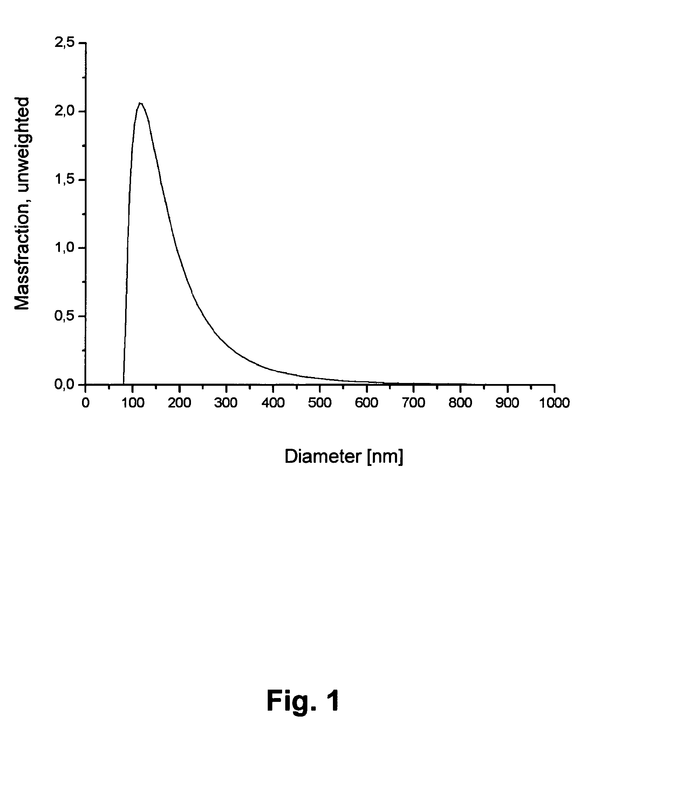

Fig. 1 shows the size distribution of nanoparticles produced by solvent evaporation. For purification and separation of the polymer particles, the suspension is centrifuged. The obtained supernatant is disregarded, the pellet washed for several times in water and then re-suspended in a small volume of water. To increase purity, the centrifugation step is repeated twice. In order to obtain a freeze-dried powder of the particulate system of the invention, the pellet is re-suspended in a small amount of water and the whole suspension subsequently freeze-dried by addition of mannitol. According to Fig. 1, the size distribution peaks at a diameter of approximately 150 nm. The unweighted mass fraction at this diameter is around 2.0.

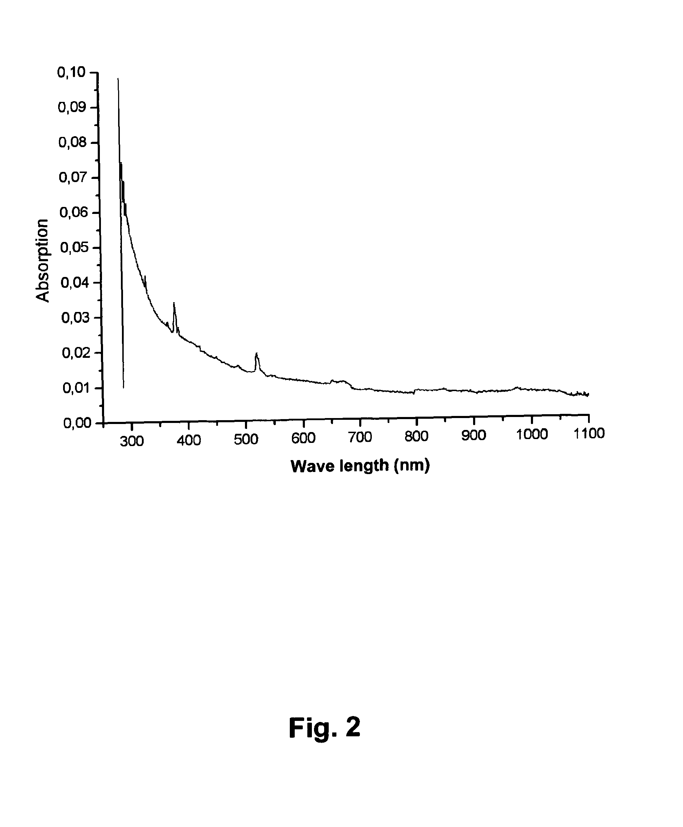

Fig. 2 shows the photometric detection of erbium in PLGA nanoparticles. The photometric determination of the erbium content is achieved by dissolving a defined amount of particles in DMF and comparison with a pure erbium-DMF solution of a known concentration. Experiments have been performed indicating that the large enrichment of the lanthanide compound in the polymer is due to intermolecular interactions. A mixture of polymer and erbium acetate in DMF was exposed to ultra filtration.

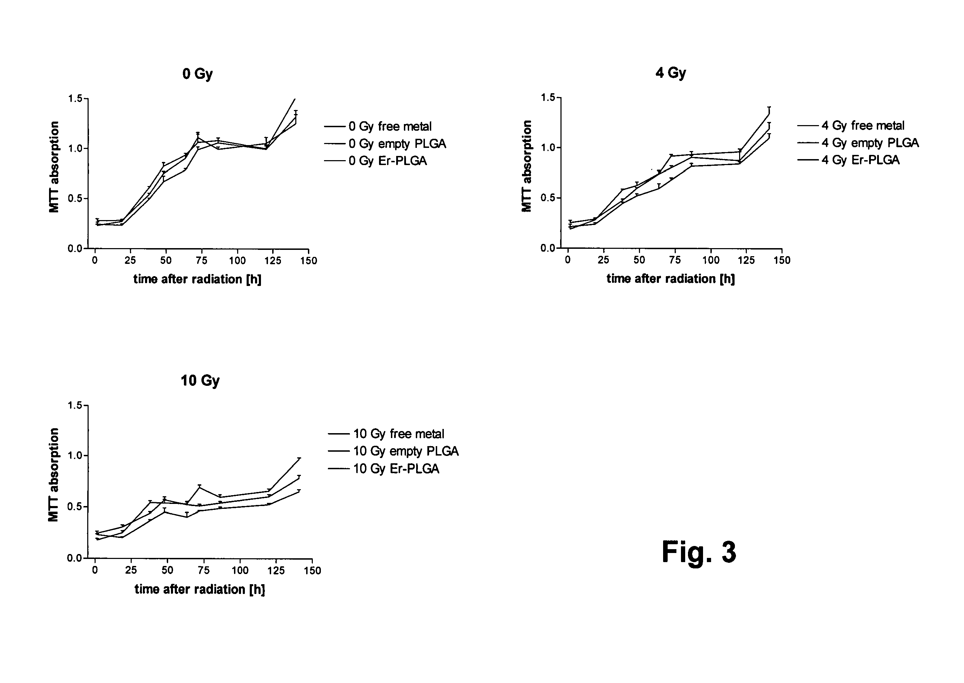

The pharmacological effect of the particulate system was investigated in cells of the lung carcinoma cell line A549. In order to determine a reduction of cell growth, A549 cells were seeded in 96 well plates and incubated for approximately 24 h before further treatments in order to reach nearly complete fixation of the cells to the bottom of the plates. The cells were then incubated together with polymer particles that were loaded with erbium for approximately 3 hours. Following exposing the cells with lanthanide particles, the cell culture plates were irradiated with different doses of irradiation. The proliferation of cells was determined by using a MTT growth assay in order to determine survival of irradiated tumor cells. The MTT assay allows the analysis of proliferation and determination of survival of cancer cells following irradiation and is based on a reduction of yellow water soluble tetra sodium salt to a purple water insoluble formazane dye by living cells. The cell proliferation over a period of 5 to 6 days following irradiation was analyzed. The results are presented in Fig. 3.

Fig. 3 shows growth curves of A549 cells following incubation with erbium nanoparticles and subsequent irradiation. Irradiation of particle treated cells was performed using a linear accelerator MD2. Already at very low doses of irradiation with 4 Gy, cell proliferation was significantly reduced using the particulate system according to the invention (ErPLGA) in comparison to the control (empty particles; empty PLGA). At higher doses, cell growth was further diminished. The experiments in Fig. 3 demonstrate that cell growth is reduced by more than 50 % after 75 h at a dose of 4 Gy using the lanthanide loaded polymer particles of the invention over the placebo control.

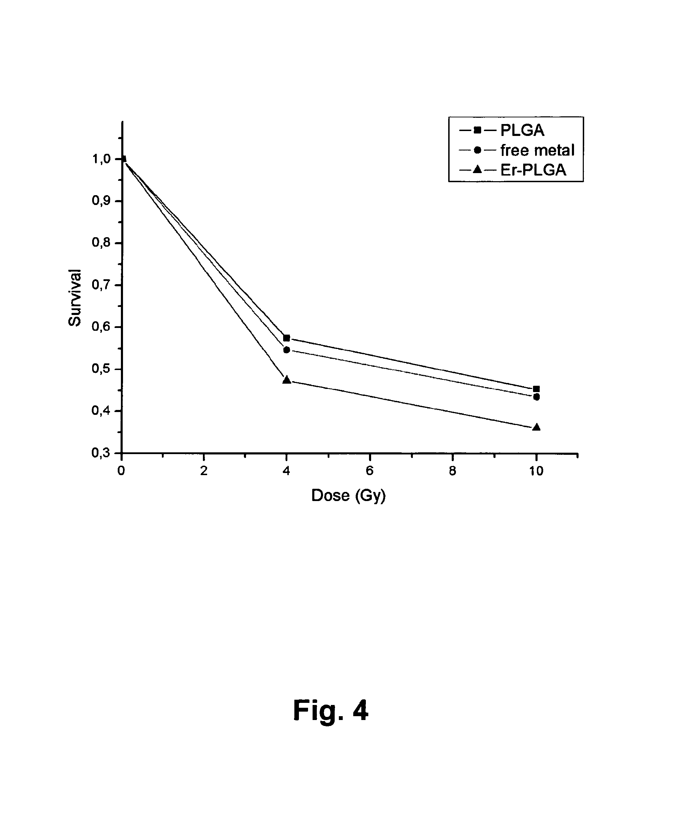

Fig. 4 shows the calculated survival of A549 cells after incubation with erbium nanoparticles and subsequent irradiation.

Survival was calculated by using a mathematical approach in which cell survival is calculated using the following formula:

$$Survival = 2 \wedge -(t_{delay} / t_{doubling\ time})$$

T doubling time = time for cell doubling
T delay = time required to achieve a specific absorption value in the MTT test of the irradiated sample in comparison to the control.

Fig. 4 shows the dose-dependant survival of A549 cells. Survival of the untreated (non-irradiated) sample was set to 100 % at 0 Gy. The survival of the placebo control (empty PLGA) is reduced with increasing doses of irradiation. When free erbium as irradiation enhancer is added, cell survival was detected to be lower, whereas after incubation of the cells with erbium loaded particles, the reduction of cell growth is significantly higher.

In order to determine lanthanide-dependent absorption properties, A549 cells were incubated with different test samples and irradiated with variable irradiation doses. Irradiation with a monochromatic pattern above and below the k absorption profile of the lanthanide was applied.

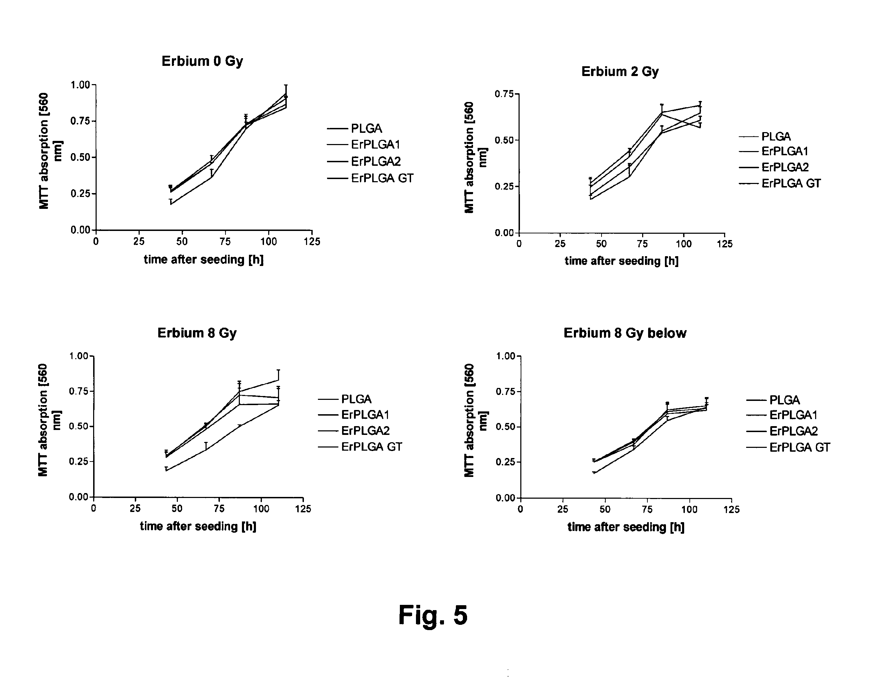

Fig. 5 shows cell growth of A549 cells after incubation with erbium nanoparticles and irradiation. PLGA = empty particle/placebo; ErPLGA1 = 0.6 mmol Er, ErPLGA2 = 1.2 mmol Er, ErPLGA GT = freeze-dried sample.

According to Fig. 5, cell growth is significantly diminished using ErPLGA particles in comparison with a control (empty particles). Surprisingly, the freeze-dried sample (ErPLGA GT) exhibits the strongest effect.

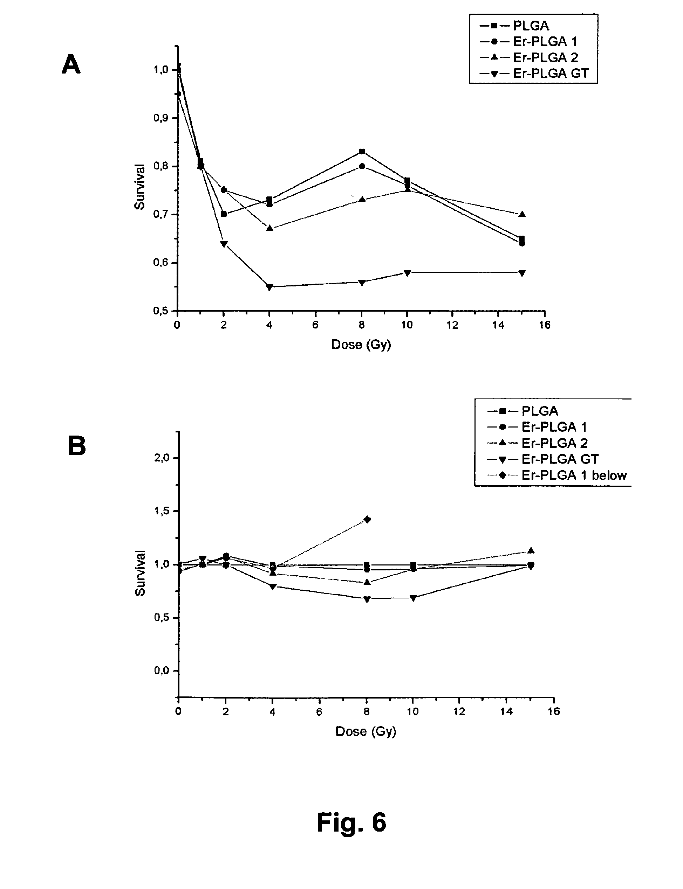

In Fig. 6, cell survival against increasing irradiation doses is demonstrated. In the experiments to Fig. 6A, survival of the placebo control of the non-irradiated sample was set to 100 %. In Fig. 6B, survival of the placebo control was set to 100 % for the respective irradiation dose.

According to Fig. 6, survival was significantly reduced using the particulate system of the invention as compared to the placebo control. The irradiation effect is dose-dependent, and peaks at 4 to 8 Gy. At higher irradiation doses, cell death is observed. The irradiation effect is dependent on the utilized lanthanide and the excitation frequency. Irradiation at wave lengths below the absorption profile of erbium, for instance, results in a survival similar to the control (Fig. 6B).

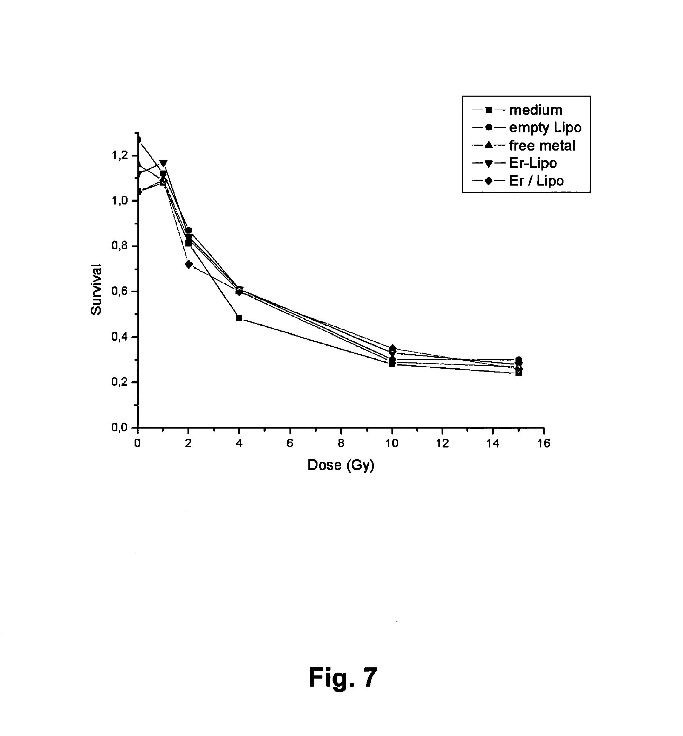

Fig. 7 is a comparative example that shows dose-dependent survival using liposomes as carrier for the lanthanide (erbium) instead of the solid polymer particle according to the invention. Fig. 7 clearly shows that there is no significant difference in survival after irradiation. Therefore, the particulate system of the invention has significant advantages over a liposome-based system.

**Claims**

1. A particulate system for use in diminishing cell growth, in particular the growth of cancer cells, comprising one or more water soluble lanthanide compounds that are embedded in a solid biodegradable polymer particle, the polymer being selected from the group consisting of polycarbonic acids, polylactic acids, polyglycolic acids, polypeptides or combinations thereof.

2. The particulate system according to claim 1, wherein the lanthanide compounds are selected from the group consisting of lanthanum, cerium, praseodymium, neodymium, promethium, samarium, europium, gadolinium, erbium, dysprosium, holmium, erbium, thulium, ytterbium, lutetium, scandium, yttrium.

3. The particulate system according to claim 1 or claim 2, wherein the lanthanide compound is a lanthanide salt.

4. The particulate system according to any one of claims 1 to 3, wherein the polymer is selected from the group consisting of all optically active (D-/L-/DL-) forms of poly(glycolic acid) (PGA), poly(lactic acid) (PLA), poly (lactide-co-glycolide) copolymers (PLGA), polydioxanone (PDS), polyacrylates, polyketales, polycyanoacrylates, polyorthoesters, polyacetates, poly(ε caprolactone), polyphosphozene, polycarbonates, polypeptides, polyiminocarbonates, poly(β-hydroxyester).

5. The particulate system according to any one of claims 1 to 4, wherein the polymer has a free carboxylic acid end group, an ester terminated end group, or an alkyl ester end group.

6. The particulate system according to any one of claims 1 to 5, wherein the polymer is poly(D,L-lactide-co-glycolide) with a free carboxylic acid end group.

7. The particulate system according to any one of claims 1 to 6, wherein the lanthanide loaded polymer particles are obtained by solvent evaporation.

8. The particulate system according to any one of claims 1 to 7, wherein the polymer particle further contains an other irradiation enhancer and/or cytostatic,

9. The particulate system according to any one of claims 1 to 8, wherein the polymer particles loaded with one or more lanthanide compounds are provided in the form freeze-dried powder.

10. A method for diminishing cell growth, comprising the steps of exposing cells to a particulate system, comprising one or more water soluble lanthanide compounds as irradiation enhancer, wherein the lanthanide compounds are embedded in a biodegradable polymer particle as carrier, the polymer being selected from the group consisting of polycarbonic acids, polylactic acids, polyglycolic acids, polypeptides or combinations thereof, and exposing the cells that are treated with the particulate system to irradiation at a wave length that results in an excitation of the lanthanide compound(s).

11. The method according to claim 10, wherein the irradiation dose for treating the cells that were exposed to lanthanide loaded polymer particles with irradiation is at least 4 Gy.

12. The method according to any one of claims 10 and 11, wherein the pre-incubation time for the irradiation treatment of the cells that were exposed to lanthanide loaded polymer particles is at least 24 h.

13. A method for producing polymer particles, comprising one or more water soluble lanthanide compounds that are embedded in a solid biodegradable polymer particle, the polymer being selected from the group consisting of polycarbonic acids, polylactic acids, polyglycolic acids, polypeptides or combinations thereof by incubating the polymer with the lanthanide compound in a suitable solvent solution, emulsifying the polymer/lanthanide mixture and applying solvent evaporation to hardening the particles.

14. A pharmaceutical composition, comprising one or more water soluble lanthanide compounds that are embedded in a solid biodegradable polymer particle, the polymer being selected from the group consisting of polycarbonic acids, polylactic acids, polyglycolic acids, polypeptides or combinations thereof for use in the treatment of a pathological disease.

15. The pharmaceutical composition according to claim 14, wherein the pathological disease is a bacterial or fungal infection, or cancer.

**Fig. 1**

**Fig. 2**

Fig. 3

8

**Fig. 4**

Fig. 5

Fig. 6

**Fig. 7**

**EP 2 567 702 A1**

Europäisches Patentamt
European Patent Office
Office européen des brevets

# EUROPEAN SEARCH REPORT

Application Number

EP 11 00 7401

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/121631 A2 (FRAUNHOFER GES FORSCHUNG [DE]; YISSUM RES DEVELOPEMENT COMPAN [IL]; BO) 8 October 2009 (2009-10-08) | 1-7, 13-15 | INV. A61K33/00 A61K9/16 A61P35/00 A61P31/10 A61P31/04 |
| Y | * page 28, paragraph 4; claims 19,21,23-25, 43-45 * * page 25, paragraph 2 * | 1-15 | |
| Y | US 2003/049202 A1 (DE STASIO GELSOMINA [US] ET AL) 13 March 2003 (2003-03-13) * the whole document * | 1-15 | |
| A | US 2006/204445 A1 (ATALA ANTHONY [US] ET AL) 14 September 2006 (2006-09-14) * paragraphs [0083], [0120]; claims 1-3, 8-9 * | 1-15 | |
| | | | TECHNICAL FIELDS SEARCHED (IPC) A61K A61P |

The present search report has been drawn up for all claims

| Place of search | Date of completion of the search | Examiner |
|---|---|---|
| Munich | 17 February 2012 | Ansaldo, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 11 00 7401

This annex lists the patent family members relating to the patent documents cited in the above-mentioned European search report.
The members are as contained in the European Patent Office EDP file on
The European Patent Office is in no way liable for these particulars which are merely given for the purpose of information.

17-02-2012

| Patent document cited in search report | Publication date | Patent family member(s) | Publication date |
|---|---|---|---|
| WO 2009121631 A2 | 08-10-2009 | EP 2106806 A1<br>EP 2257312 A2<br>JP 2011516443 A<br>US 2011064652 A1<br>WO 2009121631 A2 | 07-10-2009<br>08-12-2010<br>26-05-2011<br>17-03-2011<br>08-10-2009 |
| US 2003049202 A1 | 13-03-2003 | NONE | |
| US 2006204445 A1 | 14-09-2006 | US 2006204445 A1<br>WO 2006099373 A2 | 14-09-2006<br>21-09-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6770020 B2

- US 5888997 A

**Non-patent literature cited in the description**

- **T. NAWROTH et al.** Synchrotron Irradiation in Material Sciences. *SRMS 4, Conference,* 23 August 2004